(19) Europäisches Patentamt
European Patent Office
Office européen des brevets

(11) **EP 4 659 666 A1**

(12) **EUROPEAN PATENT APPLICATION**

(43) Date of publication:
**10.12.2025 Bulletin 2025/50**

(21) Application number: **25179132.3**

(22) Date of filing: **27.05.2025**

(51) International Patent Classification (IPC):
**A61B 5/08** (2006.01)    **A61B 5/11** (2006.01)
**A61B 5/00** (2006.01)

(52) Cooperative Patent Classification (CPC):
**A61B 5/0816; A61B 5/1128; A61B 5/4809;
A61B 5/4812; A61B 5/7267**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **03.06.2024   US 202463655473 P
12.05.2025   US 202519205787**

(71) Applicant: **Covidien LP
Mansfield, MA 02048 (US)**

(72) Inventors:
• **Addison, Anthony P.**
  **Mansfield, 02048 (US)**
• **Montgomery, Dean**
  **Mansfield, 02048 (US)**
• **Addison, Paul S.**
  **Mansfield, 02048 (US)**

(74) Representative: **Hargreaves, Timothy Edward
Marks & Clerk LLP
40 Torphichen Street
Edinburgh EH3 8JB (GB)**

(54)    **DETERMINATION OF SLEEP STATES USING MACHINE LEARNING**

(57)    Implementations described herein disclose a method including determining, based on an image signal received from a camera focused on at least a portion of a patient, a respiratory waveform, receiving an observed sleep signal of the patient temporally corresponding to the respiratory waveform, the observed sleep signal including sleep state labels, labeling various segments of the respiratory waveform using sleep-wake state labels to generate a labeled respiratory waveform, generating an input feature matrix by processing the labeled respiratory waveform, and training a machine learning (ML) model using the input feature matrix.

FIG. 3

EP 4 659 666 A1

# Description

## Cross-Reference to Related Applications

This application claims priority and the benefit of U.S. Provisional Patent Application No. 63/655,473, filed June 3, 2024, and U.S. Patent Application No. 19/205,787, filed May 12, 2025, the entire disclosures of which are incorporated herein by reference in their entirety.

## Background

Sleep is a vital activity that every organism needs to function properly. The lack of sleep or poor sleep patterns can have significant impacts on a variety of essential day-to-day functions. Most humans experience various states of sleep during their sleep cycle. Specifically, the sleep cycle of humans includes rapid eye movement sleep (REMS), non-rapid eye movement sleep (N-REMS) state. Furthermore, the N-REMS state includes N1, N2, and N3 states, with each state leading to progressively deeper sleep. The percentage of time a human spends in one of these sleep states depends on a number of factors, including health, age, etc. The neural activity of humans during each of the states is also different and the amount of time in a particular state affects the amount of rest received from sleep and the health benefits therefrom.

## Summary

Sleep is a vital activity that every organism needs to function properly. The lack of sleep or poor sleep patterns can have significant impacts on a variety of essential day-to-day functions. Most humans experience various states of sleep during their sleep cycle. Specifically, the sleep cycle of humans includes rapid eye movement sleep (REMS), non-rapid eye movement sleep (N-REMS) state. Furthermore, the N-REMS state includes N1, N2, and N3 states, with each state leading to progressively deeper sleep. The percentage of time a human spends in one of these sleep states depends on a number of factors, including health, age, etc. The neural activity of humans during each of the states is also different and the amount of time in a particular state affects the amount of rest received from sleep and the health benefits therefrom. Implementations described herein disclose a method including determining, based on an image signal received from a camera focused on at least a portion of a patient, a respiratory waveform, receiving an observed sleep signal of the patient temporally corresponding to the respiratory waveform, the observed sleep signal including sleep state labels, labeling various segments of the respiratory waveform using sleep-wake state labels to generate a labeled respiratory waveform, generating an input feature matrix by processing the labeled respiratory waveform, and training a machine learning (ML) model using the input feature matrix.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

Other implementations are also described and recited herein.

## Brief Descriptions of the Drawings

A further understanding of the nature and advantages of the present technology may be realized by reference to the figures, which are described in the remaining portion of the specification.

FIG. 1 illustrates an example implementation of a system for determining sleep state for a patient using machine learning as disclosed herein.

FIG. 2 illustrates example waveforms of various physiological signals indicating sleep state for a patient, including the output of the system for determining sleep states using machine learning as disclosed herein.

FIG. 3 illustrates example operations of the system for determining sleep state for a patient using machine learning as disclosed herein.

FIG. 4 illustrates alternative example operations of the system for determining sleep state for a patient using machine learning as disclosed herein.

FIG. 5 shows an example machine learning (ML) model used by the system for determining sleep state for a patient using machine learning as disclosed herein.

FIG. 6 shows a portable non-contact subject monitoring system that includes a non-contact detector and a computing device.

FIG. 7 shows a semi-portable non-contact subject monitoring system that includes a non-contact detector and a computing device.

FIG. 8 shows a non-portable non-contact subject monitoring system that includes a non-contact detector and a computing device.

FIG. 9 is a block diagram illustrating a system including a computing device, a server, and an image capture device.

## Detailed Descriptions

Sleep is a vital activity that every organism needs to function properly. The lack of sleep or poor sleep patterns can have significant impacts on a variety of essential day-to-day functions. Most humans experience various states of sleep during their sleep cycle. Specifically, the sleep cycle of humans includes rapid eye movement sleep (REMS), non-rapid eye movement sleep (N-REMS) state. Furthermore, the N-REMS state includes N1, N2, and N3 states, with each state leading to progressively deeper sleep. The percentage of time a human spends in one of these sleep states depends on a number of factors, including health, age, etc. The neural activity of humans during each of the states is also different and the amount of time in a particular state affects the amount of rest received from sleep and the health benefits therefrom.

It is important for a healthcare provider to determine the quality and amount of sleep that a patient gets. One measure used to determine the quality of sleep is the apnea-hypopnea index (AHI). The AHI is a scale that tells whether the patient has a sleep disorder called apnea and, if so, how serious it is. Apneas indicate temporary cessation of breathing by a patient. Sleep apnea is a potentially serious sleep disorder indicating a condition during which the patient's breathing repeatedly stops and starts. Hypopnea is a condition that indicates reduction in the level of breathing by the patient. The AHI may be calculated as a total number of apnea and hypopnea events during sleep divided by the total sleep time (TST).

The technology disclosed herein provides a touchless system for detecting and displaying respiratory events during the sleep cycle of patients using a respiratory waveform that is derived from depth images. Specifically, a camera is used to generate a series of depth images of a patient during the sleep cycle, such as overnight. The depth images are processed to generate a respiratory waveform. Subsequently, portions of the respiratory waveform are labeled with electroencephalogram (EEG)-based sleep state classifications derived during the sleep cycle. EEG-based sleep state classification may identify sleep states as one of five sleep states, namely wakefulness (W), non-rapid eye movement (NREM) sleep state 1 (N1), NREM sleep state 2 (N2), NREM sleep state 3 (N3) and rapid eye movement (REM) sleep state.

In one implementation, the respiratory waveform labeled with the sleep state classifications is used to train a machine learning (ML) model. In an alternative implementation, the respiratory waveform labeled with the sleep state classifications is further processed to generate an input feature matrix. Subsequently, the input feature matrix is used to train the ML model. In another implementation, the input feature matrix may be generated directly from the depth image signal received from a camera. In another implementation, the sequence of depth images may be used directly to train the ML model.

Once the ML model is trained, a section of real-time respiratory waveform may be input into the trained ML model to generate prediction of sleep states during the time covered by the real-time respiratory waveform section. Alternatively, if the input feature matrix used to train the ML model were generated directly from the depth image signal received from a camera, a section of real-time depth image signal received from a camera may be input into the trained ML model to generate prediction of sleep states during the time covered by the real-time depth image signal section.

The system for determining sleep states as disclosed herein can be used to replace the existing systems for determining sleep states that uses wired probes to collect signals from the users. As a result, the system disclosed herein is much less intrusive to the patients, especially when the patients are sleeping or trying to sleep. Alternatively, implementations of the system for determining sleep states as disclosed herein may also be used for home baby monitoring to automatically determine total sleep time and sleep/wake patterns of infants, which may be related to their sleep quality and/or used to determine best feeding times.

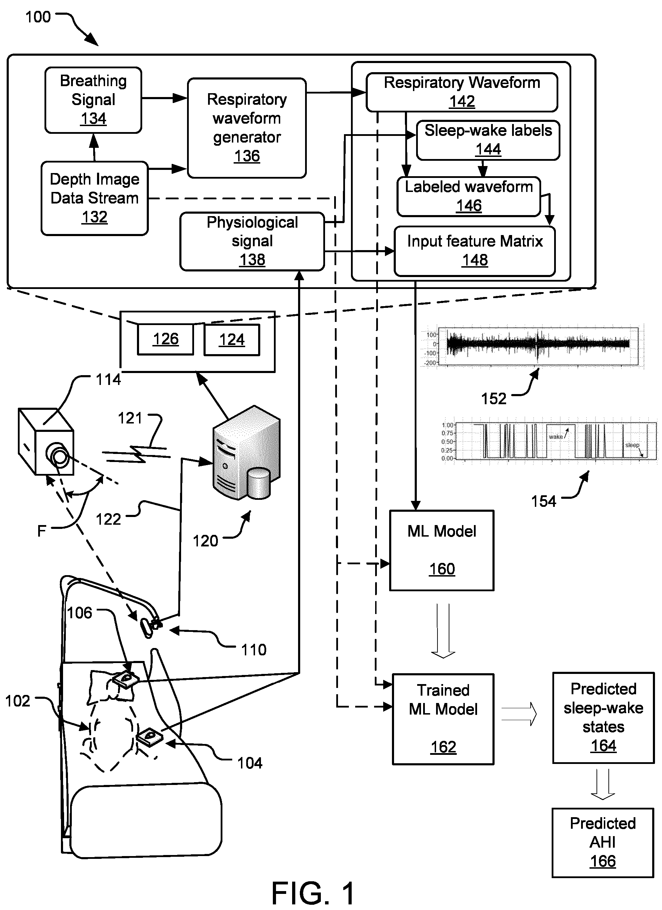

FIG. 1 illustrates an implementation of a sleep state determination system 100 for determining sleep state for a patient 102 using machine learning as disclosed herein. The system 100 includes a non-contact detector system 110 placed remote from the patient 102. In this embodiment, the detector system 110 includes a camera system 114, particularly, a camera that may include an infrared (IR) detection feature. The camera system 114 may be a depth sensing camera system, such as a Kinect camera from Microsoft Corp. (Redmond, Washington) or a RealSense™ D415, D435 or D455 camera from Intel Corp. (Santa Clara, California).

The camera system 114 is remote from the patient 102, in that it is spaced apart from and does not physically contact the patient 102. The camera system 114 may be positioned in close proximity to or on the bed of the patient 102. The camera system 114 has a field of view F that encompasses at least a portion of the patient 102. The field of view F may be selected to be at least the torso of the patient 102. The camera system 114 includes a depth sensing camera that can detect a distance between the camera system 114 and objects in its field of view F. Such information can be used to determine that the patient 102 is within the field of view of the camera system 114 and determine a region of interest (ROI) to monitor on the subject. The ROI may be the entire field of view F or may be less than the entire field of view F. Once an ROI is identified, the distance to the desired feature is determined and the desired measurement(s) can be made.

The camera system 114 is remote from the patient 102, in that it is spaced apart from and does not physically contact the patient 102. The camera system 114 may be positioned in close proximity to or on the bed of the patient 102. The camera system 114 has a field of view F that encompasses at least a portion of the patient 102. The field of view F may be selected to be at least the torso of the patient 102. The camera system 114 includes a depth sensing camera that can detect a distance between the camera system 114 and objects in its field of view F. Such information can be used to determine that the patient 102 is within the field of view of the camera system 114 and determine a region of interest (ROI) to monitor on the subject. The ROI may be the entire field of view F or may be less than the entire field of view F. Once an ROI is identified, the distance to the desired feature is determined and the desired measurement(s) can be made.

The measurements (e.g., one or more of depth signal, RGB reflection, light intensity) are sent to a computing device 120 through a wired or wireless connection 121. Alternatively, a wired connection 122 from the non-contact detector system 110 may communicate the signals to the computing device 120. The computing device 120 includes a processor 124 and memory 126 for storing data, software, computer instructions, etc. Sequential image frames of the patient 102 are recorded by the video camera system 114 and sent to the computing device 120 for analysis by the processor 124. Other embodiments of the computing device 120 may have different, fewer, or additional components than shown in FIG. 1. In some embodiments, the computing device 120 may be a server. In other embodiments, the computing device 120 of FIG. 1 may be connected to a server. The captured images (e.g., still images or video) can be processed or analyzed at the computing device 120 and/or at the server to create a topographical map or image to identify the patient 102 and any other objects within the ROI.

The signals collected from the camera system 114 may be stored in the memory 126. For example, the signals from the camera system 114 may be stored as a depth image data stream 132. For example, the depth image data stream 132 may include depth of frames as captured by the camera system. Alternatively, the depth image data stream 132 may include depth of frames in the form or RGB signals, infra-red (IR) signals, etc.

Furthermore, the memory 126 may store various computer programs, software, instructions, etc., to process the data including the depth image data stream 132. In one implementation, the depth image data stream 132 may be processed to generate a breathing signal 134. The breathing signal 134 may be in the form of a flow signal representing the volume of breathing by the patient 102 measured in terms of ml of air breathed over a period such as ml/sec. Alternatively, the breathing signal 134 may be in the form of a volume signal generated as an integral of the flow signal over a segment of time.

The memory 126 may further include a respiratory waveform generator 136 including various computer executable instructions to generate a respiratory waveform 142. In one implementation, the respiratory waveform generator 136 may use both the breathing signal 134 as well as the depth image data stream to generate the respiratory waveform 142. An example of the respiratory waveform is depicted by a graph 152 indicating respiratory flow rate in mL/second over time. The memory 126 may also store a physiological signal 138 for the patient 102, where the physiological signal 138 may be collected from an EEG sensor 106. For example, the physiological signal 138 may be an electroencephalography (EEG) signal 138 that measures brain activity of the patient 102. The EEG signal 138 may be used to generate sleep states of the patient 102. For each of the sleep states, awake, REM, N1, N2, N3, sleep-wake labels 144 may be generated and stored in the memory 126. An example of sleep-wake labels 144 is illustrated by a graph 154 where the wake state is indicated by one (1) and a sleep state is indicated by zero (0).

Alternative implementation may have the sleep-wake labels 144 generated based on an alternative physiological signal such as photoplethysmographic (PPG) signal, electroencephalography (EEG) signal, electrocardiography (ECG) signal, electromyography (EMG) signal, accelerometry signal, pressure signal, peripheral oxygen saturation (SpO2) signal, heart rate (HR), etc.

Subsequently, a labeled waveform 146 may be generated where various section of the respiratory waveform 142 are labeled with sleep-wake labels 144. Specifically, such labeled waveform 146 may be in the form of the respiratory waveform 142 where various sections of the respiratory waveform 142 are labeled with the sleep state. In one implementation, an input feature matrix 148 is generated by processing the labeled respiratory waveform 146. Such processing of the labeled waveform 146 may include, filtering the labeled respiratory waveform 146 to remove high or low frequency noise outside of the expected respiratory frequency range. This may be achieved using a bandpass filter. Alternatively, the labeled respiratory waveform 146 may be filtered to remove mean of the labeled respiratory waveform 146 to center the labeled respiratory waveform 146 around zero.

In one implementation, the labeled respiratory waveform 146 may be processed to scale the labeled respiratory waveform 146 such that the maximum and the minimum excursions of the labeled respiratory waveform are set to limits such as +1 and -1, respectively. Alternatively, the labeled respiratory waveform 146 maybe transformed using, for example, a Fourier transform to provide frequency-based information as input. In another implementation, a time-frequency transform such as a STFT or wavelet transform may be applied to the labeled respiratory waveform 146 to generate the input feature matrix 148. In this case the input feature matrix 148 may be in the form of a two-dimensional (time-frequency) matrix of values.

The processing of the labeled respiratory waveform 146 may also include down sampling the input waveform. For example, the data used to generate the respiratory waveform 142 may be collected at 30 Hz, but the labeled respiratory waveform 146 may be down sampled to 10Hz or 5Hz. By doing this, fewer parameters are required in the network and may make the training of the ML model 160 as well as generating inferences using the trained model 162 more efficient without any loss in performance.

A segment of the selected input signal, such as the labeled respiratory waveform 146, the input feature matrix 148, the depth data stream 132, or other selected input signal is used to train the ML model 160. For example, such segment of the input signal may be for example, 20s, 30s, 40s, 50s, 100s, 250s, 350s, etc. long. Here the length of the segment may be limited by the lowest frequency that needs to be detected. For example, the system may be designed so that each segment contains at least one or more breaths. Thus, if the lowest respiratory rate that is desired to be detected is 6 breaths per minute, i.e., 10 second breaths, the input segment length may be selected to be 30 seconds to detect three breaths (3 * 10 = 30 seconds), 20 seconds to detect two breaths, etc.

In general, during sleep cycles the patient spend majority of the time sleeping, therefore, in the data over the sleep cycle exhibits class imbalance between the sleep states (majority class) compared to the awake states (minority class). Therefore, there are many times more sleep labels than wake labels. In one implementation, the labeled respiratory waveform 146 may be processed to remove such class imbalance between the time the patient is asleep compared to the awake time. Example rebalancing may include repeating the minority class, under sampling the majority class, repeating the minority class but with additional data augmentation, applying a weighting factor to each class in the loss function of the deep learning model (to down weight the majority class and up weight the minority class), etc. Such rebalancing improves the training and performance of the ML model 160. Furthermore, in another implementation, demographic data of the patient 102, such as age, weight, sex, etc., may also be used as part of the input feature matrix 148 to allow the ML model to differentiate between different breathing patterns of the patients based on their age, weight, etc.

In an alternative implementation, the sensor 104 may be a pulse oximeter and the physiological signal 138 may be a PPG signal. Furthermore, the input feature matrix 148 that is used to train the ML model 160 may include the PPG signal. Alternatively, the input feature matrix 148 may include the depth image data stream 132 and the pulse oximeter data in the form of the physiological signal 138. In one implementation, the PPG signal may be used to implement transfer learning in that if much more PPG signal is available than depth images, the system may train the ML model first with the PPG signal, and then fine-tune on the smaller amount of depth images. Alternatively, the ML model may be trained on the PPG signal and then fine-tuned using the PPG signal + Depth images.

In another implementation, the system 100 may implement data augmentation by training the ML model 160 with Depth images + PPG signals, however, for some percentage of the time (e.g. 5%, 10%, 25%, etc.) remove the PPG signal and for some percentage of the time remove the depth images (however, never both at the same time). Such data augmentation forces the ML model 160 to handle cases of missing data, either the PPG signal or the depth images, and makes the ML model more robust.

Alternatively, the input feature matrix 148 may include the labeled respiratory waveform 146 and the pulse oximeter data. Yet alternatively, the sensor 110 may be an oximeter and the physiological signal 138 may be the oxygen saturation level. In such implementation, the input feature matrix 148 used to train the ML model 160 may be the oxygen saturation level data $SpO_2$ and the labeled respiratory waveform 146. Yet alternatively, the sensor 110 may be a device to measure the patient's heart rate and the physiological signal 138 may be the heart rate signal. In such implementation, the input feature matrix 148 used to train the ML model 160 may be the heart rate signal and the labeled respiratory waveform 146. Yet alternatively, combination of all of the heart rate signal, the SpO2 level, and the labeled respiratory waveform 146 may be used to generate the input feature matrix 148.

Subsequently, such input feature matrix 146 may be used to train an ML model 160. Alternatively, each of the respiratory waveform 142 and the sleep-wake labels 144 temporally corresponding to the respiratory waveform 142 may be input into the ML model 160 for training. Specifically, the ML model 160 may be trained to predict sleep states based on the respiratory waveform 142.

Alternatively, the ML model 160 may be trained using the depth image data stream 132 and the sleep-wake labels 144 temporally corresponding to the depth image data stream 132. In such implementation, the ML model 160 may be trained to predict sleep states based on the depth image data stream 132. The ML model 160 may be a CNN-based ML model, an RNN-based ML model, a deep learning ML model using U-Net architecture, etc. A trained ML model 162, trained using any of the above method, may receive a real-time observed input signal to generate predicted sleep states 164. For example, such observed input signal may be the depth image data stream 132, the respiratory waveform 142, etc. The predicted sleep states 164 may indicate, at various points over a sleep cycle, whether the patient was in a wake state, in REM state, or in one of the NREM states N1, N2, and N3.

In one implementation, the predicted sleep states 164 may be used to calculate predicted apnea-hypopnea index (AHI) 166 for the patient. The AHI 166 is a scale that tells whether the patient has a sleep disorder called apnea and, if so, how serious it is. Apneas indicate temporary cessation of breathing by a patient. Specifically, the AHI 166 may be calculated as total number of apnea and hypopnea events divided by the total sleep time (TST). In the illustrated implementation, the TST may be determined based on the predicted sleep states 164 to include only the sleep states (REM and NREM (N1, N2, and N3)), while not including the wake time in the calculation of the TST.

Thus, as disclosed herein, AHI is determined as

follows:

$$AHI = (N_{apnea} + N_{hypopnea}) / TST$$

Where

     $N_{apnea}$ = number of apneas exhibited while in the sleep state (TST),
     $N_{hypopnea}$ = number of apneas exhibited while in the sleep state (TST), and
     TST = total sleep time (including REM and NREM states).

Specifically, the TST as used herein does not include the time during the awake state, thus providing more accurate value of the AHI.

Implementations of the system for determining sleep states as disclosed herein may also be deployed at home using only a depth camera and a wrist-based wearable oximeter for $SpO_2$ and HR determination. The data may be wirelessly communicated to a remote system that deploys a trained ML model to infer sleep states for the patient and the resulting AHI.

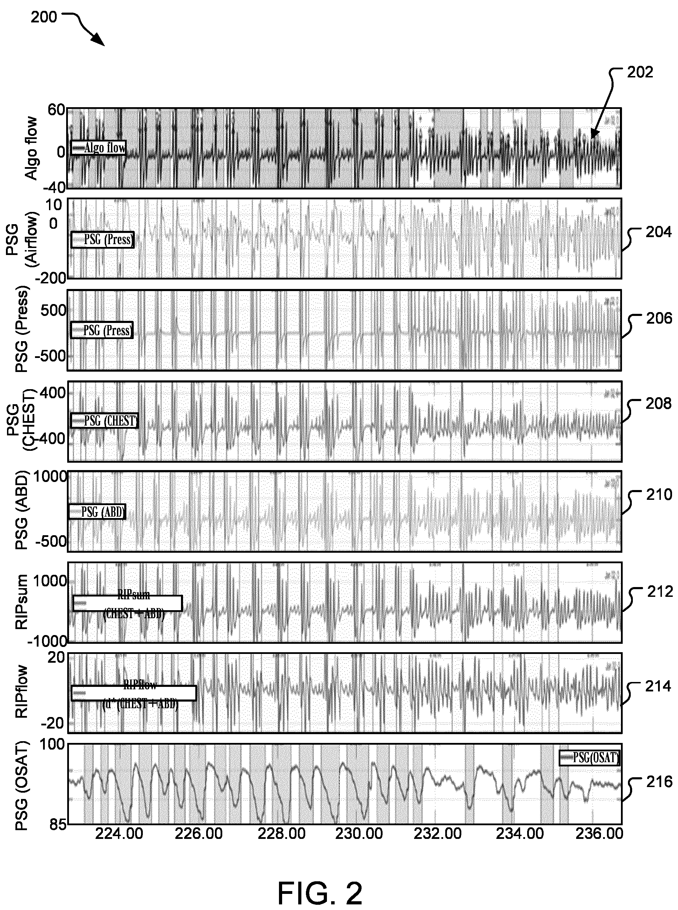

FIG. 2 illustrates waveforms 200 of various physiological signals indicating sleep state for a patient. Specifically, the graph 202 illustrates a graph of respiratory events detected during sleep together with a respiratory waveform 202 derived from depth images received from a touchless patient monitoring system.

The graph 204 illustrates nasal airflow measurements during PSG on the patient, whereas graph 206 illustrates nasal pressure measurements during PSG on the patient. The graph 208 illustrates RIP chest signal 208 generated using a RIP chest band on chest of the patient, whereas the graph 210 illustrates RIP abdomen signal 210 generated using a RIP abdomen band on abdomen of the patient. The graph 212 illustrates PSG RIPsum, which is the sum of the RIP chest signal 208 and the RIP abdomen signal 210.

The graph 214 illustrates a PSG flow signal which is a derivative of the PSG RIPsum signal 212. Finally, 216 illustrates an oxygen saturation ($SpO_2$) signal indicating oxygen saturation (OSAT) levels of the patient.

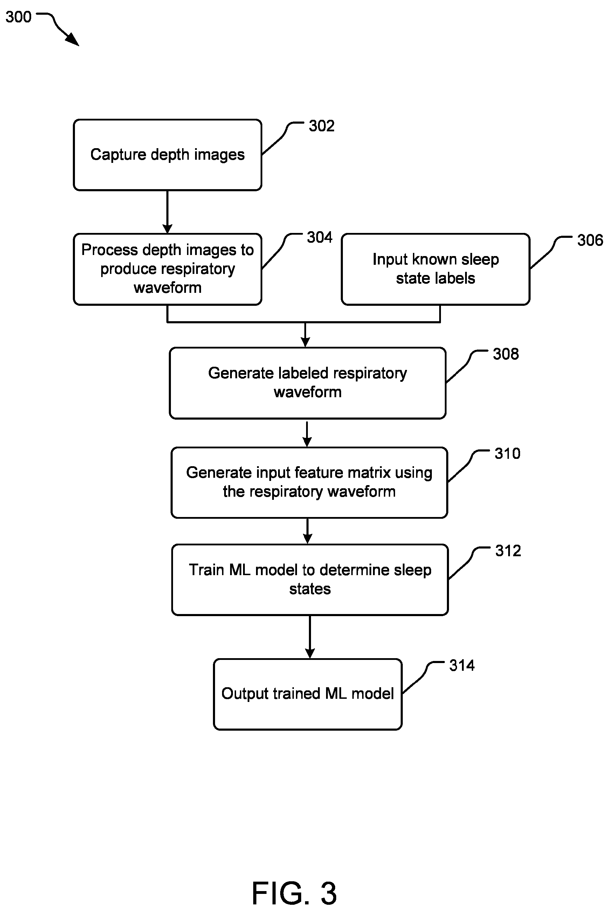

FIG. 3 illustrates operations 300 of the system for determining sleep state for a patient using machine learning as disclosed herein.

An operation 302 captures depth images of a patient during the patient's sleep cycle. For example, such depth images may be captured by a camera, such as the camera 114 disclosed in FIG. 1. An operation 304 processes the depth images to produce a respiratory waveform. An operation 306 inputs known sleep state labels which may be used at operation 308 to label the respiratory waveform to generate a labeled respiratory waveform. Subsequently, an operation 310 processes the labeled respiratory waveform to generate an input feature matrix for training an ML model.

An operation 312 trains the ML model using the input feature matrix. For example, the input feature matrix over a number of different sleep cycles may be input into the ML model to train the ML model to determine sleep states based on the respiratory waveform. An operation 314 outputs the trained ML model that may be used with real-time input signals to inference sleep states for a patient. Examples of the real-time signals may include respiratory waveform, depth images, etc.

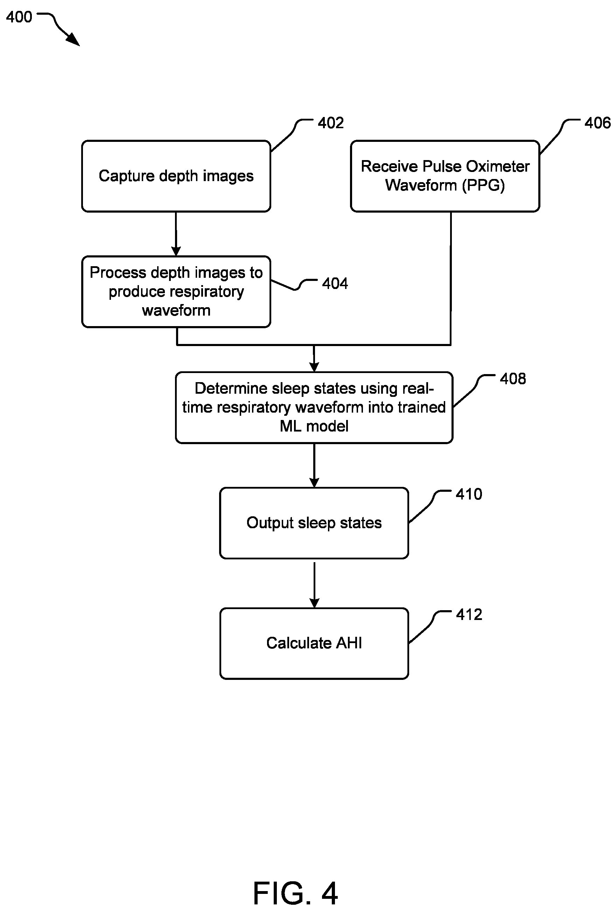

FIG. 4 illustrates alternative operations 400 of the system for determining sleep state for a patient using machine learning as disclosed herein. An operation 402 captures depth images of a patient during the patient's sleep cycle. For example, such depth images may be captured by a camera, such as the camera 114 disclosed in FIG. 1. An operation 404 processes the depth images to produce a respiratory waveform. An operation 406 receives pulse oximetry (PPG) waveform.

At operation 408, the respiratory waveform and the PPG data may be input into an ML model that is trained to determine sleep states using the respiratory waveform and the PPG data. At operation 410, the trained ML model outputs the sleep states over the sleep cycle. An operation 412 calculates the AHI of the patient using the TST calculated based on the sleep states of the patient as provided by operation 410.

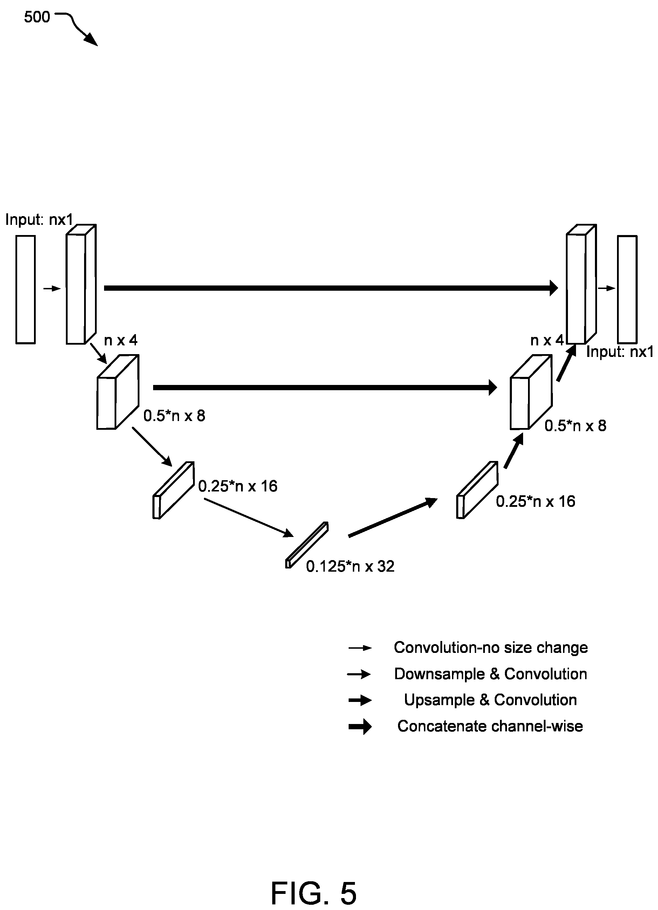

FIG. 5 shows architecture of a machine learning (ML) model 500 used by the system for determining sleep state for a patient using machine learning as disclosed herein. Specifically, the ML model 500 uses U-Net architecture that is used for semantic segmentation of images. Detecting the wake/sleep state is effectively a segmentation of a time series signal. Using a U-Net model allows predicting a sequence instead of a single value for a window. Specifically, the U-Net model represents a class of networks and maybe configured in a number of different shapes.

While the implementation disclosed herein uses U-Net architecture, alternative implementation of the AI model 500 may use a CNN-based model (e.g. based on a ResNet architecture), a long-short term memory (LSTM) model, a Transformer or any other suitable ML model. For example, alternative ML models may use an RNN-based model including one of the following: Simple RNN, a GRU (Gated Recurrent Unit). In addition, the AI model may use CNN components and RNN components together.

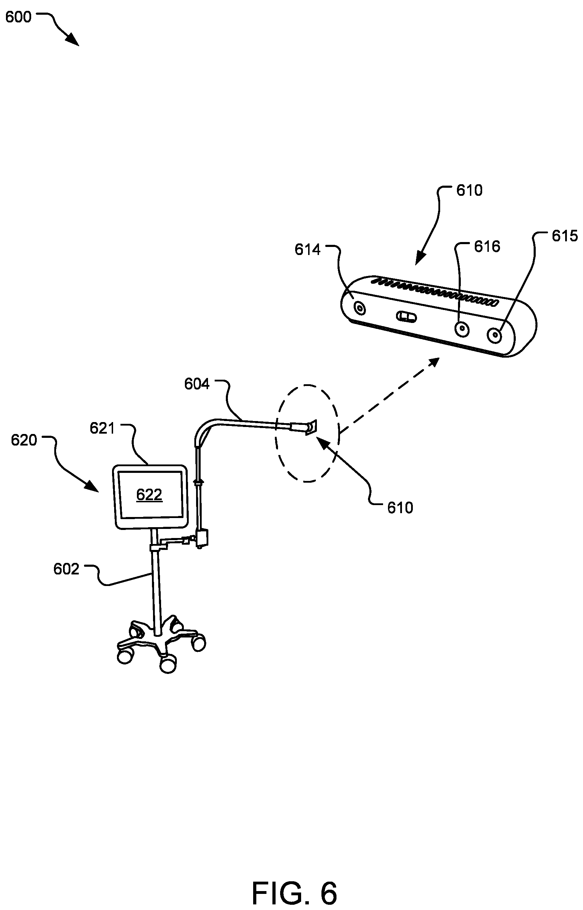

FIG. 6 shows a portable non-contact subject monitoring system 600 that includes a non-contact detector 610 and a computing device 620. In this embodiment, the non-contact detector 610 and the computing device 620 are generally fixed in relation to each other and the system 600 is readily moveable in relation to the subject to be monitored. The detector 610 and the computing device 620 are supported on a trolley or stand 602, with the detector 610 on an arm 604 that is pivotable in relation to the stand 602 as well as adjustable in height. The system 600 can be readily moved and positioned where desired.

The detector 610 includes a first camera 614 and a second camera 615, at least one of which includes an infrared (IR) camera feature. The detector 610 also includes an IR projector 616, which projects individual features (e.g., dots, crosses or Xs, lines, or a featureless pattern, or a combination thereof etc.).

The detector 610 may be wired or wireless connected to the computing device 620. The computing device 620 includes a housing 621 with a touch screen display 622, a processor (not seen), and hardware memory (not seen) for storing software and computer instructions.

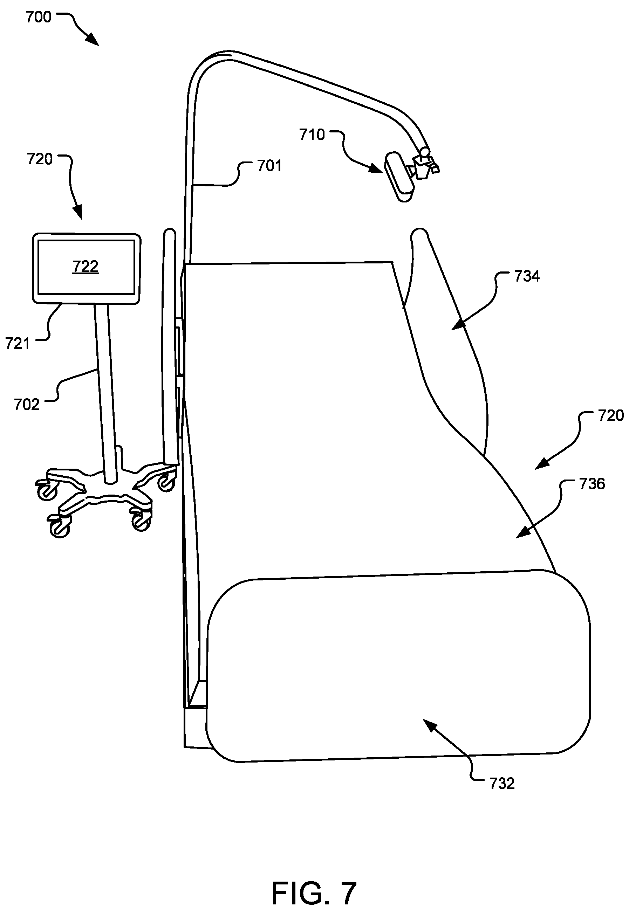

FIG. 7 shows a semi-portable non-contact subject monitoring system 700 that includes a non-contact detector 710 and a computing device 720. In this embodiment, the non-contact detector 710 is in a fixed relation to the subject to be monitored and the computing device 720 is readily moveable in relation to a subject lying on a bed 730. Specifically, the bed 730 may have a headboard 732, a side rail 734, and a mattress 736.

The detector 710 is supported on an arm 701 that is attached to a bed, in this embodiment, a hospital bed, although the detector 710 and the arm 701 can be attached to a crib, a bassinette, an incubator, an isolette, or other bed-type structure. In some embodiments, the arm 701 is pivotable in relation to the bed as well as adjustable in height to provide for proper positioning of the detector 710 in relation to the subject.

The detector 710 may be wired or wireless connected to the computing device 720, which is supported on a moveable trolley or stand 702. The computing device 720 includes a housing 721 with a touch screen display 722, a processor (not seen), and hardware memory (not seen) for storing software and computer instructions.

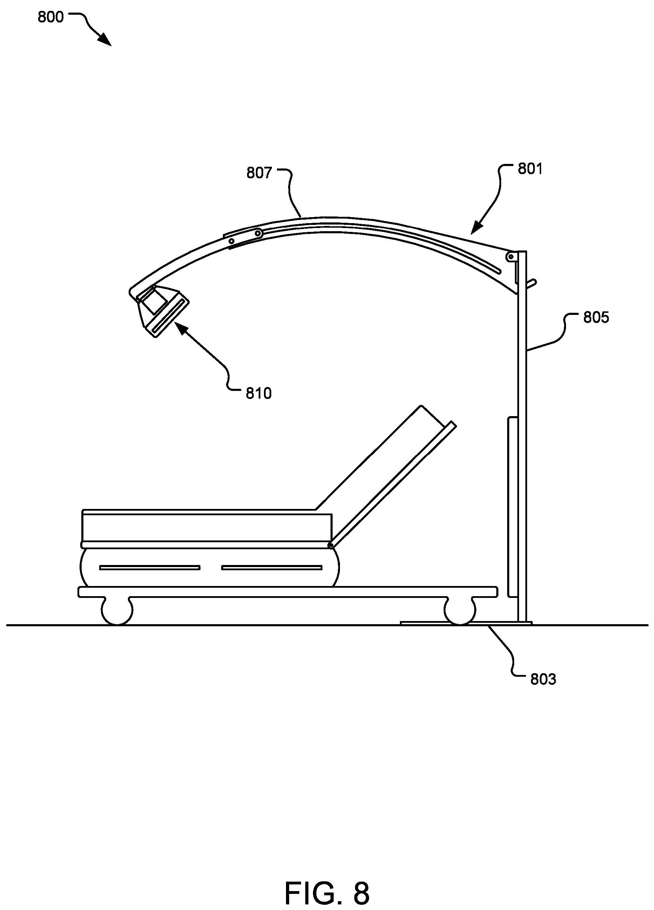

FIG. 8 shows a non-portable non-contact subject monitoring system 800 that includes a non-contact detector 810 and a computing device (not seen in FIG. 8). In this embodiment, at least the non-contact detector 810 is generally fixed in a location, configured to have the subject to be monitored moved into the appropriate position to be monitored.

The detector 810 is supported on a stand 801 that is free standing, the stand having a base 803, a frame 805, and a gantry 807. The gantry 807 may have an adjustable height, e.g., movable vertically along the frame 805, and may be pivotable, extendible and/or retractable in relation to the frame 805. The stand 801 is shaped and sized to allow a bed or bed-type structure to be moved (e.g., rolled) under the detector 810.

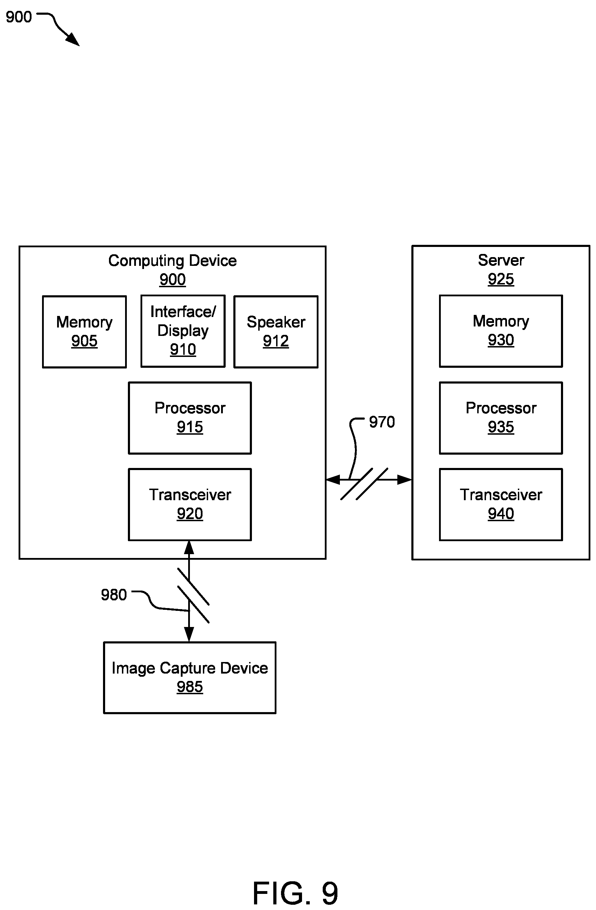

FIG. 9 is a block diagram illustrating a system including a computing device 900, a server 925, and an image capture device 985 (e.g., a camera, e.g., the camera system 114). In various embodiments, fewer, additional and/or different components may be used in the system.

The computing device 900 includes a processor 915 that is coupled to a memory 905. The processor 915 can store and recall data and applications in the memory 905, including applications that process information and send commands/signals according to any of the methods disclosed herein. In one implementation, the memory 905 may be non-transitory computer readable memory configured to store various computer-executable instructions that are executable on the processor or processor unit 935. Alternatively, the memory 905 may be physical article of manufacture that includes one or more computer-readable storage media encoding computer-executable instructions for executing on a computer system a computer process. The processor 915 may also display objects, applications, data, etc. on an interface/display 910 and/or provide an audible alert via a speaker 912. The processor 915 may also or alternately receive inputs through the interface/display 910. The processor 915 is also coupled to a transceiver 920. With this configuration, the processor 915, and subsequently the computing device 900, can communicate with other devices, such as the server 925 through a connection 970 and the image capture device 985 through a connection 980. For example, the computing device 900 may send to the server 925 information determined about a subject from images captured by the image capture device 985, such as depth information of a subject or object in an image.

The server 925 also includes a processor 935 that is coupled to a memory 930 and to a transceiver 940. The processor 935 can store and recall data and applications in the memory 930. With this configuration, the processor 935, and subsequently the server 925, can communicate with other devices, such as the computing device 900 through the connection 970.

The computing device 900 may be, e.g., the computing device 120 of FIG. 1. Accordingly, the computing device 900 may be located remotely from the image capture device 985, or it may be local and close to the image capture device 985 (e.g., in the same room). The processor 915 of the computing device 900 may perform any or all of the various steps disclosed herein. In other embodiments, the steps may be performed on a processor 935 of the server 925. In some embodiments, the various steps and methods disclosed herein may be performed by both of the processors 915 and 935. In some embodiments, certain steps may be performed by the processor 915 while others are performed by the processor 935. In some embodiments, information determined by the processor 915 may be sent to the server 925 for storage and/or further processing.

The devices shown in the illustrative embodiment may be utilized in various ways. For example, either or both of the connections 970, 980 may be varied. For example, either or both the connections 970, 980 may be a hard-wired connection. A hard-wired connection may involve connecting the devices through a USB (universal serial bus) port, serial port, parallel port, or other type of wired connection to facilitate the transfer of data and information between a processor of a device and a second processor of a second device. In another example, one or both of the connections 970, 980 may be a dock where one device may plug into another device. As another example, one or both of the connections 970, 980 may be a wireless connection. These connections may be any sort of wireless connection, including, but not limited to, Bluetooth connectivity, Wi-Fi connectivity, infrared, visible light, radio frequency (RF) signals, or other wireless protocols/methods. For example, other possible modes of wireless communication may include near-field communications, such as passive radio-frequency identification (RFID) and active RFID technologies. RFID and similar near-field communications may allow the various devices to communicate in short range when they are placed proximate to one another. In yet another example, the various devices may connect through an internet (or other network) connection. That is, one or both of the connections 970, 980 may represent several different computing devices and network components that allow the various devices to communicate through the internet, either through a hard-wired or wireless connection. One or both of the connections 970, 980 may also be a combination of several modes of connection.

The configuration of the devices in FIG. 9 is merely one physical system on which the disclosed embodiments may be executed. Other configurations of the devices shown may exist to practice the disclosed embodiments. Further, configurations of additional or fewer devices than the ones shown in FIG. 9 may exist to practice the disclosed embodiments. Additionally, the devices shown in FIG. 9 may be combined to allow for fewer devices than shown or separated such that more than the three devices exist in a system. It will be appreciated that many various combinations of computing devices may execute the methods and systems disclosed herein. Examples of such computing devices may include other types of infrared cameras/detectors, night vision cameras/detectors, other types of cameras, radio frequency transmitters/receivers, smart phones, personal computers, servers, laptop computers, tablets, RFID enabled devices, or any combinations of such devices.

In contrast to tangible computer-readable storage media, intangible computer-readable communication signals may embody computer readable instructions, data structures, program modules or other data resident in a modulated data signal, such as a carrier wave or other signal transport mechanism. The term "modulated data signal" means a signal that has one or more of its characteristics set or changed in such a manner as to encode information in the signal. By way of example, and not limitation, intangible communication signals include wired media such as a wired network or direct-wired connection, and wireless media such as acoustic, RF, infrared and other wireless media.

The implementations described herein are implemented as logical steps in one or more computer systems. The logical operations may be implemented (1) as a sequence of processor-implemented steps executing in one or more computer systems and (2) as interconnected machine or circuit modules within one or more computer systems. The implementation is a matter of choice, dependent on the performance requirements of the computer system being utilized. Accordingly, the logical operations making up the implementations described herein are referred to variously as operations, steps, objects, or modules. Furthermore, it should be understood that logical operations may be performed in any order, unless explicitly claimed otherwise or a specific order is inherently necessitated by the claim language.

The above specification, examples, and data provide a complete description of the structure and use of exemplary embodiments of the invention. Since many implementations of the invention can be made without departing from the spirit and scope of the invention, the invention resides in the claims hereinafter appended. Furthermore, structural features of the different embodiments may be combined in yet another implementation without departing from the recited claims.

The following examples are illustrative of the techniques described herein.

Example 1. A method, comprising: determining, based on an image signal received from a camera focused on at least a portion of a patient, a respiratory waveform; receiving an observed sleep signal of the patient temporally corresponding to the respiratory waveform, the observed sleep signal including sleep state labels; labeling various segments of the respiratory waveform using sleep-wake state labels to generate a labeled respiratory waveform; generating an input feature matrix by processing the labeled respiratory waveform; and training a machine learning (ML) model using the input feature matrix.

Example 2. The method of Example 1, wherein labeling various segments of the respiratory waveform using sleep-staging labels further comprising labeling various segments of the respiratory waveform using electroencephalogram (EEG) based sleep-staging labels.

Example 3. The method of Example 1, wherein labeling various segments of the respiratory waveform using sleep-staging labels further comprising labeling various segments of the respiratory waveform using Photoplethysmography (PPG) based sleep-staging labels.

Example 4. The method of Example 1, further comprising: inputting a real-time respiratory waveform into the trained ML model to generate inferred sleep/-wake states of the patient.

Example 5. The method of Example 1, further comprising: inputting the image signal received from a camera into the trained ML model to generate inferred sleep/wake states of the patient.

Example 6. The method of Example 1, further comprising receiving a PPG signal of the patient, wherein training a machine learning (ML) model further comprising training the ML model using combination of the PPG signal and the input feature matrix.

Example 7. The method of Example 6, wherein training a machine learning (ML) model further comprising training the ML model using only one of the PPG signal and the input feature matrix for at least some portion of time.

Example 8. The method of Example 1, wherein processing the labeled respiratory waveform further comprising transforming the labeled respiratory waveform using a Fourier transform to provide frequency-based information as input.

Example 9. The method of Example 1, wherein processing the labeled respiratory waveform further comprising processing the labeled respiratory waveform such that the maximum and the minimum excursions of the labeled respiratory waveform are set to limits such as +1 and -1, respectively.

Example 10. A system comprising: memory; one or more processor units; a sleep state determination system stored in the memory and executable by the one or more processor units, the sleep state determination encoding computer-executable instructions on the memory for executing on the one or more processor units a computer process, the computer process comprising: determining, based on an image signal received from a camera focused on at least a portion of a patient, a respiratory waveform; receiving an observed sleep signal of the patient temporally corresponding to the respiratory waveform, the observed sleep signal including sleep state labels; labeling various segments of the respiratory waveform using sleep-wake state labels to generate a labeled respiratory waveform; generating an input feature matrix by processing the labeled respiratory waveform; and training a machine learning (ML) model using the input feature matrix.

Example 11. The system of Example 10, wherein labeling various segments of the respiratory waveform using sleep-staging labels further comprising labeling various segments of the respiratory waveform using electroencephalogram (EEG) based sleep-staging labels.

Example 12. The system of Example 10, wherein labeling various segments of the respiratory waveform using sleep-staging labels further comprising labeling various segments of the respiratory waveform using Photoplethysmography (PPG) based sleep-staging labels.

Example 13. The system of Example 10, further comprising receiving a PPG signal of the patient, wherein training a machine learning (ML) model further comprising training the ML model using combination of the PPG signal and the input feature matrix.

Example 14. The system of Example 13, wherein training a machine learning (ML) model further comprising training the ML model using only one of the PPG signal and the input feature matrix for at least some portion of time.

Example 15. The system of Example 10, wherein processing the labeled respiratory waveform further comprising transforming the labeled respiratory waveform using a Fourier transform to provide frequency-based information as input.

Example 16. A physical article of manufacture including one or more tangible computer-readable storage media encoding computer-executable instructions for executing on a computer system a computer process to determine respiratory rate of a patient, the computer process comprising: determining, based on an image signal received from a camera focused on at least a portion of a patient, a respiratory waveform; receiving an observed sleep signal of the patient temporally corresponding to the respiratory waveform, the observed sleep signal including sleep state labels; labeling various segments of the respiratory waveform using sleep-wake state labels to generate a labeled respiratory waveform; generating an input feature matrix by processing the labeled respiratory waveform; and training a machine learning (ML) model using the input feature matrix.

Example 17. The physical article of manufacture of Example 16, wherein labeling various segments of the respiratory waveform using sleep-staging labels further comprising labeling various segments of the respiratory waveform using electroencephalogram (EEG) based sleep-staging labels.

Example 18. The physical article of manufacture of Example 16, wherein processing the labeled respiratory waveform further comprising transforming the labeled respiratory waveform using at least one of Fourier transform to provide frequency-based information as input and a time-frequency transform.

Example 19. The physical article of manufacture of Example 16, further comprising inputting a real-time respiratory waveform into the trained ML model to generate inferred sleep/wake states of the patient.

Example 20. The physical article of manufacture of Example 16, wherein labeling various segments of the respiratory waveform using sleep-staging labels further comprising labeling various segments of the respiratory waveform using Photoplethysmography (PPG) based sleep-staging labels.

**Claims**

1. A method, comprising:

   determining (304, 404), based on an image signal received from a camera (114) focused on at least a portion of a patient, a respiratory waveform;
   receiving an observed sleep signal of the patient temporally corresponding to the respiratory waveform, the observed sleep signal including sleep state labels (144);

labeling (308) various segments of the respiratory waveform using sleep-wake state labels to generate a labeled respiratory waveform; generating (310) an input feature matrix by processing the labeled respiratory waveform; and training (312) a machine learning (ML) model (160) using the input feature matrix.

2. The method of claim 1, wherein labeling various segments of the respiratory waveform using sleep-staging labels further comprising labeling various segments of the respiratory waveform using electroencephalogram (EEG) based sleep-staging labels.

3. The method of claim 1, wherein labeling various segments of the respiratory waveform using sleep-staging labels further comprising labeling various segments of the respiratory waveform using Photoplethysmography (PPG) based sleep-staging labels.

4. The method of claims 1, 2, or 3, further comprising: inputting a real-time respiratory waveform into the trained ML model to generate inferred sleep/wake states of the patient.

5. The method of claims 1, 2, or 3, further comprising: inputting the image signal received from a camera into the trained ML model to generate inferred sleep/-wake states of the patient.

6. The method of claim 1, further comprising receiving a PPG signal of the patient, wherein training a machine learning (ML) model further comprising training the ML model using combination of the PPG signal and the input feature matrix.

7. The method of claim 6, wherein training a machine learning (ML) model further comprising training the ML model using only one of the PPG signal and the input feature matrix for at least some portion of time.

8. The method of claims 1, 2, or 3, wherein processing the labeled respiratory waveform further comprising transforming the labeled respiratory waveform using a Fourier transform to provide frequency-based information as input.

9. The method of claim 1, wherein processing the labeled respiratory waveform further comprising processing the labeled respiratory waveform such that the maximum and the minimum excursions of the labeled respiratory waveform are set to limits such as +1 and - 1, respectively.

10. A system comprising:

memory;
one or more processor units;

a sleep state determination system stored in the memory and executable by the one or more processor units, the sleep state determination encoding computer-executable instructions on the memory for executing on the one or more processor units a computer process, the computer process comprising:

determining (304, 404), based on an image signal received from a camera (114) focused on at least a portion of a patient, a respiratory waveform; receiving an observed sleep signal of the patient temporally corresponding to the respiratory waveform, the observed sleep signal including sleep state labels (144); labeling (308) various segments of the respiratory waveform using sleep-wake state labels to generate a labeled respiratory waveform; generating (310) an input feature matrix by processing the labeled respiratory waveform; and training (312) a machine learning (ML) model (160) using the input feature matrix.

11. The system of claim 10, wherein labeling various segments of the respiratory waveform using sleep-staging labels further comprising labeling various segments of the respiratory waveform using electroencephalogram (EEG) based sleep-staging labels.

12. The system of claim 10, wherein labeling various segments of the respiratory waveform using sleep-staging labels further comprising labeling various segments of the respiratory waveform using Photoplethysmography (PPG) based sleep-staging labels.

13. The system of any one of claims 10-12, further comprising receiving a PPG signal of the patient, wherein training a machine learning (ML) model further comprising training the ML model using combination of the PPG signal and the input feature matrix.

14. The system of claim 13, wherein training a machine learning (ML) model further comprising training the ML model using only one of the PPG signal and the input feature matrix for at least some portion of time.

15. A physical article of manufacture including one or more tangible computer-readable storage media encoding computer-executable instructions for executing on a computer system a computer process to determine respiratory rate of a patient, the computer process comprising:

determining (304, 404), based on an image signal received from a camera (114) focused on at least a portion of a patient, a respiratory waveform;

receiving an observed sleep signal of the patient temporally corresponding to the respiratory waveform, the observed sleep signal including sleep state labels (144);

labeling (308) various segments of the respiratory waveform using sleep-wake state labels to generate a labeled respiratory waveform;

generating (310) an input feature matrix by processing the labeled respiratory waveform; and training (312) a machine learning (ML) model (160) using the input feature matrix.

FIG. 1

FIG. 2

300

```
┌─────────────────────┐
│  Capture depth images│ ─── 302
└─────────────────────┘
          │
          ▼
┌─────────────────────┐              ┌─────────────────────┐
│ Process depth images │ ─── 304      │  Input known sleep   │ ─── 306
│  to produce respiratory│            │   state labels       │
│     waveform         │              └─────────────────────┘
└─────────────────────┘
          │                                    │
          └────────────────┬───────────────────┘
                           ▼
              ┌─────────────────────────┐
              │ Generate labeled respiratory│ ─── 308
              │      waveform           │
              └─────────────────────────┘
                           │
                           ▼
              ┌─────────────────────────┐
              │ Generate input feature matrix using│ ─── 310
              │   the respiratory waveform │
              └─────────────────────────┘
                           │
                           ▼
              ┌─────────────────────────┐
              │ Train ML model to determine sleep│ ─── 312
              │          states          │
              └─────────────────────────┘
                           │
                           ▼
              ┌─────────────────────────┐
              │  Output trained ML model │ ─── 314
              └─────────────────────────┘
```

# FIG. 3

400

402 Capture depth images

406 Receive Pulse Oximeter Waveform (PPG)

404 Process depth images to produce respiratory waveform

408 Determine sleep states using real-time respiratory waveform into trained ML model

410 Output sleep states

412 Calculate AHI

# FIG. 4

500

Input: nx1

n x 4

0.5*n x 8

0.25*n x 16

0.125*n x 32

n x 4

Input: nx1

0.5*n x 8

0.25*n x 16

→ Convolution-no size change

→ Downsample & Convolution

➡ Upsample & Convolution

➡ Concatenate channel-wise

FIG. 5

600

610

614

616

615

604

621

620

622

602

610

FIG. 6

FIG. 7

800

807    801

810

805

803

FIG. 8

900

Computing Device
900

Memory
905

Interface/
Display
910

Speaker
912

Processor
915

Transceiver
920

970

Server
925

Memory
930

Processor
935

Transceiver
940

980

Image Capture Device
985

FIG. 9

| | Europäisches Patentamt |
|---|---|
| | European Patent Office |
| | Office européen des brevets |

## EUROPEAN SEARCH REPORT

**Application Number**

EP 25 17 9132

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JONATHAN CARTER ET AL: "Deep Learning-Enabled Sleep Staging From Vital Signs and Activity Measured Using a Near-Infrared Video Camera", ARXIV.ORG, CORNELL UNIVERSITY LIBRARY, 201 OLIN LIBRARY CORNELL UNIVERSITY ITHACA, NY 14853, 6 June 2023 (2023-06-06), XP091532086, * page 3, column 1, line 3 - line 10; figure 5 * * page 4, column 1, line 9 - line 14 * * page 5, column 1, line 1 - page 5, column 2, line 33 * * page 8, column 1, line 27 - line 33 * ----- | 1-15 | INV. A61B5/08 A61B5/11 A61B5/00 |
| X | US 2023/263465 A1 (JORGE JOAO GONCALO MALVEIRO [GB] ET AL) 24 August 2023 (2023-08-24) * paragraph [0008] - paragraph [0021]; figure 1 * * paragraph [0151] * ----- | 1-15 | |
| | | | TECHNICAL FIELDS SEARCHED (IPC) |
| X | WO 2023/278319 A1 (JACKSON LAB [US]; UNIV PENNSYLVANIA [US]) 5 January 2023 (2023-01-05) * page 22, line 4 - line 8 * * page 32, line 10 - page 34, line 29; figure 1 * ----- -/-- | 1-15 | A61B |

The present search report has been drawn up for all claims

| Place of search | Date of completion of the search | Examiner |
|---|---|---|
| The Hague | 7 August 2025 | Bourquin, Yannyk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 25 17 9132

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Han Jianan ET AL: "Seeing your sleep stage: cross-modal distillation from EEG to infrared video", arXiv.org, 1 August 2022 (2022-08-01), XP093301236, Retrieved from the Internet: URL:https://arxiv.org/pdf/2208.05814 [retrieved on 2022-08-11] * page 1, column 2, line 15 - page 4, column 1, line 37; figures 1,3 * ----- | 1-15 | |
| A | CORONEL CARMINA ET AL: "3D Camera and Pulse Oximeter for Respiratory Events Detection", IEEE JOURNAL OF BIOMEDICAL AND HEALTH INFORMATICS, IEEE, PISCATAWAY, NJ, USA, vol. 25, no. 1, 20 April 2020 (2020-04-20) , pages 181-188, XP011829227, ISSN: 2168-2194, DOI: 10.1109/JBHI.2020.2984954 [retrieved on 2021-01-04] * abstract; figures 1,2 * ----- | 1-15 | |

TECHNICAL FIELDS
SEARCHED (IPC)

The present search report has been drawn up for all claims

| Place of search | Date of completion of the search | Examiner |
|---|---|---|
| The Hague | 7 August 2025 | Bourquin, Yannyk |

EPO FORM 1503 03.82 (P04C01)

## ANNEX TO THE EUROPEAN SEARCH REPORT
## ON EUROPEAN PATENT APPLICATION NO.

EP 25 17 9132

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2025

| Patent document cited in search report | Publication date | Patent family member(s) | | Publication date |
|---|---|---|---|---|
| US 2023263465 A1 | 24-08-2023 | EP | 4223214 A1 | 09-08-2023 |
| | | US | 2023263465 A1 | 24-08-2023 |
| WO 2023278319 A1 | 05-01-2023 | AU | 2022301046 A1 | 18-01-2024 |
| | | AU | 2025201972 A1 | 03-04-2025 |
| | | CA | 3224154 A1 | 05-01-2023 |
| | | CN | 117545417 A | 09-02-2024 |
| | | EP | 4340711 A1 | 27-03-2024 |
| | | JP | 2024530535 A | 22-08-2024 |
| | | KR | 20240027726 A | 04-03-2024 |
| | | US | 2024293077 A1 | 05-09-2024 |
| | | WO | 2023278319 A1 | 05-01-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63655473
- US 20578725